# EUROPEAN PATENT SPECIFICATION

(11) **EP 3 515 385 B1**
(45) Date of publication and mention of the grant of the patent: **23.02.2022**
(21) Application number: 17768796.9
(22) Date of filing: 19.09.2017
(51) Int. Cl.: A61F 13/00, A61F 13/36

(54) **METHOD FOR PRODUCING A SURGICAL GAUZE WITH A FLUORESCENT AREA.**
VERFAHREN ZUR HERSTELLUNG EINES CHIRURGISCHEN VERBANDSMULLS MIT EINEM FLUORESZENTEN BEREICH.
PROCÉDÉ DE FABRICATION D'UNE GAZE CHIRURGICALE AVEC UNE ZONE FLUORESCENTE.

(30) Priority: 22.09.2016 IT 201600095446
(43) Date of publication of application: 31.07.2019
(73) Proprietor: Zingoni, Isabella, 06034 Foligno (PG) (IT)
(72) Inventor: Zingoni, Isabella, 06034 Foligno (PG) (IT)
(74) Representative: Baldi, Claudio
(86) International application number: PCT/EP2017/073569
(87) International publication number: WO 2018/054874

(56) References cited:
- WO-A1-2014/144624
- WO-A1-2015/017044
- WO-A1-2016/077259
- US-A1- 2002 029 032
- US-A1- 2006 047 238
- US-A1- 2016 120 409

## Description

The present patent application for industrial invention relates to a method for producing a surgical gauze provided with a fluorescent area.

The peculiarities and the advantages of the present invention will be more evident after a brief description of the prior art and of its main drawback.

As it is known, hydrophilic gauze is largely used during surgical operations and medications in general, said hydrophilic gauze substantially consisting in a sterilized tissue suitable for absorbing blood.

Such a widespread use is justified by the fact that, being provided with high absorbing capabilities, the hydrophilic gauze is especially suitable for cleaning surgical incisions and wounds in general.

In such situations, in fact, hydrophilic gauzes are used to absorb the blood that comes out of wounds and surgical incisions.

In particular, the patient may bleed abundantly in case of particularly large and deep surgical incisions, requiring the use of several gauzes to clean the incision and/or tampon the hemorrhage.

Moreover, one or more gauzes often get totally soaked with the patient's blood, thus assuming the typical red color for their entire surface.

Such a case may be very dangerous, especially when a gauze that is completely soaked with blood is positioned by the surgeon, even temporarily, on the patient's surgical field, that is to say on a surface that substantially has the same red color.

In such a situation, in fact, the gauze soaked with blood may not be immediately visible, being "camouflaged" because of its red color in the surgical field.

For this reason, at the end of the operation, the surgical staff may not notice the presence of a gauze that is "forgotten" in the surgical field and may forget to extract it before starting to suture.

After suturing, said "forgotten" gauze remains inside the body of the patient.

It is worthless saying that such a foreign body is not tolerable for the human body; for this reason, as soon as the presence of the gauze is detected, or even supposed, the patient must be taken back to the operating room in order to remove the gauze.

US2002/029032 discloses surgical articles, including needles or other hardware, and surgical supplies that include an element that fluoresces upon exposure to ultraviolet light.

WO2016/077259 discloses a surgical article that comprises a material with a fluorophore chosen between a near infrared (NIR) agent or a fluorescent protein.

WO2014/144624 discloses a surgical article including an integral construction having a plurality of micro-particle taggants that are UV or radio wave detectable.

US2016120409 discloses a method for treating a wound that provides for the following steps:
- positioning a wound filler having a coating agent containing a fluorescent material proximate the wound;
- removing the wound filler after a time period;
- scanning the wound using a fluorescence scanner to determine whether a portion of the wound filler remains at the wound.

WO2015017044 discloses a gauze-like fabric comprising two strips of fluorescent material arranged at two lateral edges of the gauze. Each strip comprises an outer surface which repels fluid, i.e. strips coated with a silicone layer, so as to be impermeable to blood.

The purpose of the present invention is to prevent the risk of not detecting the presence of a hydrophilic gauze in the surgical field, in spite of being completely soaked with blood, accidentally forgetting the gauze in the surgical field before starting to suture.

In order to achieve such a purpose, some areas of a typical hydrophilic gauze are treated with inks that can be detected when exposed to UV radiations, in such a way that the areas treated with said inks are markedly fluorescent, regardless of being soaked with blood.

In fact, the presence of a similar partially fluorescent gauze on the background of the surgical field - after being exposed to a UV light - will surely and immediately attract the attention of the surgical staff at the operating table, thus avoiding the risk of forgetting the gauze inside the surgical field.

The considerable practical efficacy of the present invention is also related to the fact that the lamps used in the operating room normally produce a UV light and therefore it not necessary to provide the existing operating rooms with a specific light source in order to detect the presence of the fluorescent gauze of the invention.

Starting from a similar idea, which represents a fully satisfactory solution, another device has been adopted to additionally increase the functional efficacy of the gauze according to the present invention.

In fact, such a device guarantees an even higher visibility of the fluorescent gauze of the invention, even in the case of a gauze which is highly soaked with blood.

In such a case, such an abundant presence of blood may partially reduce the fluorescence of the gauze, thus making it less visible in the surgical field, in spite of being exposed to UV radiation.

In order to achieve the aforesaid additional purpose, the areas of the gauze that are treated with fluorescent inks are also treated with transparent silicone in order to be impermeable to blood.

In fact, such a treatment with transparent silicone makes blood soaking impossible in the areas of the gauze that are previously treated with said fluorescent inks. Being immune from blood interference, said areas will maintain a full visibility when exposed to the light of the UV lamps that are typically used in operating rooms.

It must be noted that the products used to treat the gauze of the invention have been carefully selected among the available ones because they are not harmful for human health.

Preferably, the inks that can be detected with UV radiation are the ones that are normally used in medical and ophthalmologic applications, such as for example fluorescine, whereas the silicon is the type that is normally used in orthopedic prostheses.

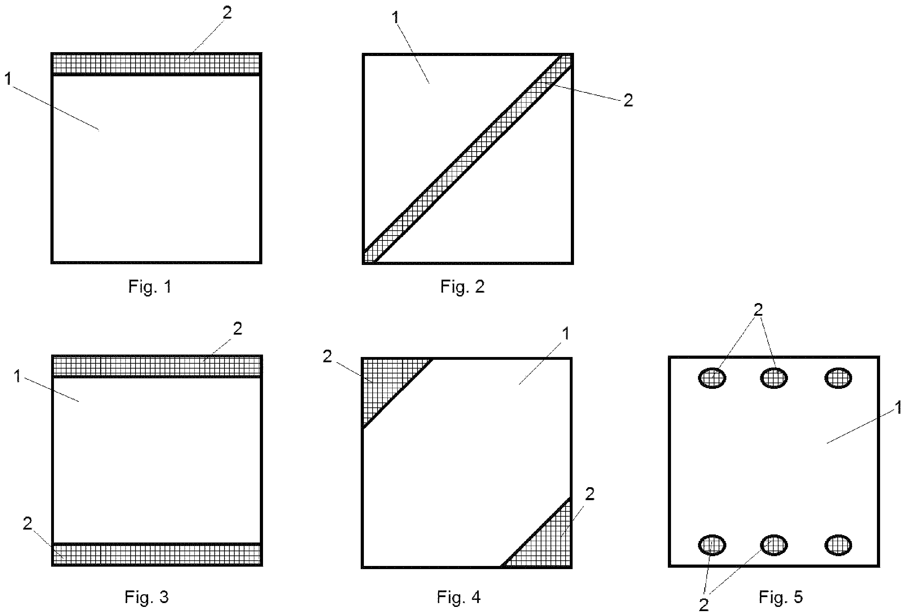

For the sake of clarity, the description of the invention continues with reference to the attached drawings, which have a merely illustrative, not limiting value, wherein Figs. 1 to 5 show some possible embodiments of a gauze obtained by the method according to the invention.

These figures show hydrophilic gauzes (1) provided with one or more areas (2) treated with fluorescent inks that are sensitive to UV light.

In particular, the embodiment of Fig. 1 is provided with a single area (2) treated with said inks, which is disposed in correspondence of one of the edges of the gauze (1).

The embodiment of Fig. 2 is provided with a single area (2) treated with said inks, which is configured as a band that extends diagonally between two corners of the gauze (1).

The embodiment of Fig. 3 is provided with two areas (2) treated with said inks, each of them being disposed in one of the edges of the gauze (1).

The embodiment of Fig. 4 is provided with two areas (2) treated with said inks, each of them being disposed in one of the angles of the gauze (1).

The embodiment of Fig. 5 is provided with six areas (2) treated with said inks, which have a substantially elliptical area and are aligned, three by three, on two rows disposed in proximity of the lateral edges of the gauze (1).

Regardless of what shown in the aforementioned illustrative figures, it is understood that said areas treated with fluorescent inks (2) may have any other position, shape and number in the gauze according to the invention (1). In particular, the gauze (1) may comprise a single area (2) treated with fluorescent ink that coincides with the entire area of the gauze.

As mentioned previously, in order to ensure a higher efficacy of the gauze of the invention (1) and, more precisely, to guarantee that its fluorescent areas (2) are well visible even if they are completely soaked with blood, each of said areas (2) is protected with transparent silicone, which makes it practically impermeable to blood.

According to an embodiment which is not part of the present invention, said fluorescent areas (2) of the gauze (1) are coated with a thin layer of silicon, which is applied during a successive production step.

According to the present invention, during the same production step, said areas (2) can be treated with a mixture formed of said fluorescent inks, with the addition of said transparent silicone.

## Claims

1. A method for producing an hydrophilic gauze comprising the following steps:
- providing a gauze;
- during the same step, treating one or more areas (2) of the gauze with a mixture formed of fluorescent UV-sensitive inks, with the addition of transparent silicone, in order to make said one or more areas (2) of the gauze impermeable to blood.

2. The method of claim 1, wherein the step of treating one or more areas (2) of the gauze with fluorescent inks with the addition of transparent silicone is applied to a single area (2) of the gauze; said single area (2) coinciding with the entire area of the gauze.

3. The method of claim 1, wherein the step of treating one or more areas (2) of the gauze with fluorescent inks with the addition of transparent silicone is applied to a single area (2) of the gauze; said single area (2) being configured as a band disposed in one edge of the gauze (1).

4. The method of claim 1, wherein the step of treating one or more areas (2) of the gauze with fluorescent inks with the addition of transparent silicone is applied to a single area (2) of the gauze; said single area (2) being configured as a diagonal band that connects two corners of the gauze (1).

5. The method of claim 1, wherein the step of treating one or more areas (2) of the gauze with fluorescent inks with the addition of transparent silicone is applied to two areas (2) of the gauze; said two areas (2) being configured as bands disposed in two edges of the gauze (1).

6. The method of claim 1, wherein the step of treating one or more areas (2) of the gauze with fluorescent inks with the addition of transparent silicone is applied to two areas (2) of the gauze; said two areas (2) being disposed in two corners of the gauze (1).

7. The method of claim 1, wherein the step of treating one or more areas (2) of the gauze with fluorescent inks with the addition of transparent silicone is applied to a plurality of areas (2) of the gauze; said areas (2) being aligned in two different rows disposed in the proximity of the side edges of the gauze (1).

## Patentansprüche

1. Verfahren zur Herstellung einer hydrophilen Gaze, umfassend folgende Schritte:
- Bereitstellen einer Gaze;
- während desselben Schritts, Behandeln eines oder mehrerer Bereiche (2) der Gaze mit einem Gemisch, gebildet aus fluoreszierenden, UVempfindlichen Tinten unter Zusatz von transparentem Silikon, um einen oder mehrere Bereiche (2) der Gaze blutundurchlässig zu machen.

2. Verfahren nach Anspruch 1, wobei der Schritt des Behandelns eines oder mehrerer Bereiche (2) der Gaze mit fluoreszierenden Tinten unter Zusatz von transparentem Silikon auf einen einzelnen Bereich (2) der Gaze angewendet wird; wobei der einzelne Bereich (2) mit dem gesamten Bereich der Gaze zusammenfällt.

3. Verfahren nach Anspruch 1, wobei der Schritt des Behandelns eines oder mehrerer Bereiche (2) der Gaze mit fluoreszierenden Tinten unter Zusatz von transparentem Silikon auf einen einzelnen Bereich (2) der Gaze angewendet wird; wobei der einzelne Bereich (2) als ein Band ausgebildet ist, das an einem Rand der Gaze (1) angeordnet ist.

4. Verfahren nach Anspruch 1, wobei der Schritt des Behandelns eines oder mehrerer Bereiche (2) der Gaze mit fluoreszierenden Tinten unter Zusatz von transparentem Silikon auf einen einzelnen Bereich (2) der Gaze angewendet wird; wobei der einzelne Bereich (2) als ein diagonales Band ausgebildet ist, das zwei Ecken der Gaze (1) verbindet.

5. Verfahren nach Anspruch 1, wobei der Schritt des Behandelns eines oder mehrerer Bereiche (2) der Gaze mit fluoreszierenden Tinten unter Zusatz von transparentem Silikon auf zwei Bereiche (2) der Gaze angewendet wird; wobei die zwei Bereiche (2) als Bänder ausgebildet sind, die an zwei Rändern der Gaze (1) angeordnet sind.

6. Verfahren nach Anspruch 1, wobei der Schritt des Behandelns eines oder mehrerer Bereiche (2) der Gaze mit fluoreszierenden Tinten unter Zusatz von transparentem Silikon auf zwei Bereiche (2) der Gaze angewendet wird; wobei die zwei Bereiche (2) in zwei Ecken der Gaze (1) angeordnet sind.

7. Verfahren nach Anspruch 1, wobei der Schritt des Behandelns eines oder mehrerer Bereiche (2) der Gaze mit fluoreszierenden Tinten unter Zusatz von transparentem Silikon auf eine Mehrzahl von Bereichen (2) der Gaze angewendet wird; wobei die zwei Bereiche (2) in zwei verschiedenen Reihen ausgerichtet sind, die nahe den Seitenrändern der Gaze (1) angeordnet sind.

## Revendications

1. Méthode pour produire une gaze hydrophile comprenant les phases suivantes :
- se fournir d'une gaze ;
- pendant cette même phase, traiter une ou plusieurs zones (2) de la gaze avec un mélange formé d'encres fluorescentes sensibles aux UV et l'ajout d'une silicone transparente pour que l'une ou plusieurs zones (2) de la gaze soient imperméables au sang.

2. Méthode selon la revendication 1, où la phase de traitement d'une ou plusieurs zones (2) de la gaze avec des encres fluorescentes et l'ajout d'une silicone transparente est appliquée sur une seule zone (2) de la gaze ; ladite seule zone (2) coïncidant avec toute la zone de la gaze.

3. Méthode selon la revendication 1, où la phase de traitement d'une ou de plusieurs zones (2) de la gaze avec des encres fluorescentes et l'ajout d'une silicone transparente est appliquée sur une seule zone (2) de la gaze ; ladite seule zone (2) étant configurée sous forme d'une bande disposée sur un bord de la gaze (1).

4. Méthode selon la revendication 1, où la phase de traitement d'une ou de plusieurs zones (2) de la gaze avec des encres fluorescentes et l'ajout d'une silicone transparente est appliquée sur une seule zone (2) de la gaze ; ladite seule zone (2) étant configurée sous forme d'une bande diagonale qui relie deux coins de la gaze (1).

5. Méthode selon la revendication 1, où la phase de traitement d'une ou de plusieurs zones (2) de la gaze avec des encres fluorescentes et l'ajout d'une silicone transparente est appliquée sur deux zones (2) de la gaze ; lesdites deux zones (2) étant configurées sous forme de bandes disposées sur deux bords de la gaze (1).

6. Méthode selon la revendication 1, où la phase de traitement d'une ou de plusieurs zones (2) de la gaze avec des encres fluorescentes et l'ajout d'une silicone transparente est appliquée sur deux zones (2) de la gaze ; lesdites deux zones (2) étant disposées sur deux coins de la gaze (1).

7. Méthode selon la revendication 1, où la phase de traitement d'une ou de plusieurs zones (2) de la gaze avec des encres fluorescentes et l'ajout d'une silicone transparente est appliquée sur une pluralité de zones (2) de la gaze ; lesdites zones (2) étant alignées sur deux rangées différentes disposées à proximité des bords latéraux de la gaze (1).
